# EUROPEAN PATENT SPECIFICATION

(11) **EP 1 058 739 B1**
(45) Date of publication and mention of the grant of the patent: **07.06.2006**
(21) Application number: 99918942.6
(22) Date of filing: 30.04.1999
(51) Int. Cl.: C12Q 1/68, C12P 19/34, G01N 1/00, C07H 21/04

(54) **METHOD FOR GENERATING GENE EXPRESSION PROFILES**
METHODE ZUR ERSTELLUNG VON GENEXPRESSIONSPROFILEN
METHODE PERMETTANT DE GENERER DES PROFILS D'EXPRESSION GENIQUE

(30) Priority: 05.11.1998 US 107248 P
(43) Date of publication of application: 13.12.2000
(73) Proprietor: Ortho-McNeil Pharmaceutical, Inc., Raritan, NJ 08869-0602 (US)
(72) Inventor: ERLANDER, Mark, G., Encinitas, CA 92024 (US); SALUNGA, Ranelle, C., San Diego, CA 92111 (US); JACKSON, Michael, R., Del Mar, CA 92014 (US); LUO, Lin, La Jolla, CA 92037 (US)
(74) Representative: Mercer, Christopher Paul
(86) International application number: PCT/US1999/009594
(87) International publication number: WO 2000/028092

(56) References cited:
- WO-A1-89/10977
- WO-A1-98/35216
- US-A- 5 859 699
- SIMONE N L ET AL: "Laser-capture microdissection: opening the microscopic frontier to molecular analysis" TRENDS IN GENETICS, ELSEVIER SCIENCE PUBLISHERS B.V. AMSTERDAM, NL, vol. 14, no. 7, 1 July 1998 (1998-07-01), pages 272-276, XP004124689 ISSN: 0168-9525
- BONNER R F ET AL: "LASER CAPTURE MICRODISSECTION: MOLECULAR ANALYSIS OF TISSUE" SCIENCE, AMERICAN ASSOCIATION FOR THE ADVANCEMENT OF SCIENCE, US, vol. 278, 21 November 1997 (1997-11-21), page 1481,1483 XP000941813 ISSN: 0036-8075
- KRIZMAN D ET AL: "Gene expression analysis of prostate cancer progression by laser capture microdissection and high density cDNA microarrays." PROCEEDINGS OF THE AMERICAN ASSOCIATION FOR CANCER RESEARCH ANNUAL, vol. 39, March 1998 (1998-03), page 453 XP002226202 89th Annual Meeting of the American Association for Cancer Research;New Orleans, Louisiana, USA; March 28-April 1, 1998, March, 1998 ISSN: 0197-016X
- GELDER VAN R N ET AL: "AMPLIFIED RNA SYNTHESIZED FROM LIMITED QUANTITIES OF HETEROGENEOUS CDNA" PROCEEDINGS OF THE NATIONAL ACADEMY OF SCIENCES OF USA, NATIONAL ACADEMY OF SCIENCE. WASHINGTON, US, vol. 87, March 1990 (1990-03), pages 1663-1667, XP002942745 ISSN: 0027-8424
- LUO L ET AL: "GENE EXPRESSION PROFILES OF LASER-CAPTURED ADJACENT NEURONAL SUBTYPES" NATURE MEDICINE, NATURE PUBLISHING, CO, US, vol. 5, no. 1, January 1999 (1999-01), pages 117-122, XP002923326 ISSN: 1078-8956
- SIMONE N. L. ET AL.: 'Laser-Capture Microdissection: Opening the Microscopic Frontier to Molecular Analysis' TIG vol. 14, no. 7, July 1998, pages 272 - 276, XP004124689
- EMMERT-BUCK M. R. ET AL.: 'Laser Capture Microdissection' SCIENCE vol. 274, 08 November 1996, pages 998 - 1001, XP002923325
- ZHAO N. ET AL: 'High-Density cDNA Filter Analysis: a Novel Approach for Large-Scale, Quantitative Analysis of Gene Expression' GENE vol. 156, 1995, pages 207 - 213, XP002094436
- SCHENA M. ET AL.: 'Quantitative Monitoring of Gene Expression Patterns with a Coplementary DNA Microarray' SCIENCE vol. 270, 20 October 1995, pages 467 - 470, XP000644675
- LUO L. ET AL.: 'Gene Expression Profiles of Laser-Captured Adjacent Neuronal Subtypes' NATURE MEDICINE vol. 5, no. 1, January 1999, pages 117 - 122, XP002923326

## Description

### BACKGROUND OF THE INVENTION

Gene expression profiles of thousands of genes can now be examined *en masse* via cDNA and oligonucleotide microarrays (reviews ¹⁻³). Recently, studies have been reported that examined gene expression changes in yeast ^{4 5} as well as in mammalian cell lines ⁶, primary cells ⁷ and tissues ⁸.

However, present applications of microarray technology do not include the study of gene expression from individual cell types residing in a given tissue/organ (i.e., *in-situ).* Such studies would greatly facilitate our understanding of the complex interactions that exist in-vivo between neighboring cell types in normal and disease states. The present invention demonstrates that gene expression profiles from adjacent cell types can be successfully obtained by integrating the technologies of laser capture microdissection ⁹ (LCM) and T7-based RNA amplification ¹⁰ with cDNA microarrays ¹¹.

Krizman et al (1998), Proceedings of the American Association for Cancer Research, 39:453, discloses a method comprising selecting and isolating cells for microdissection, extracting RNA, producing labelled cDNA, hybridising the labelled cDNA to an array of immobilized DNA probes and determining which immobilized probes hybridised to which cDNA. Similar disclosures are made in Simone et al (1998), Trends in Genetics 14:272-276 and Bonner et al (1997), Science, 278: 1481-1483. However, none of these documents disclose the amplification of RNA prior to producing the labelled cDNA.

### SUMMARY OF THE INVENTION

The present invention provides a method for the reproducible measurement and assessment of the expression of specific messenger RNA's in a specific set of cells. This method combines and utilizes the known techniques of laser capture microdissection, T-7 based RNA amplification, production of cDNA from the amplified RNA, and DNA microarrays containing immobilized DNA molecules for a wide variety of specific genes to produce a profile of gene expression analysis for very small numbers of specific cells in a new way. The desired cells are individually identified and attached to a substrate by the laser capture technique, and the captured cells are separated from the remaining cells.

RNA is then extracted from the captured cells and amplified about one million-fold using the T7-based amplification technique, and, optionally, cDNA is prepared from the amplified RNA. A wide variety of specific DNA molecules are prepared which hybridize with specific nucleic acids of interest which may or may not be present, or are present at some level in the captured cells, and the DNA molecules are immobilized on a suitable substrate to form the microarray. The cDNA made from the captured cells is applied to the microarray under conditions that allow hybridization of the cDNA to the immobilized DNA on the array. The expression profile of the captured cells is obtained from the analysis of the hybridization results using the amplified RNA or, optionally, cDNA made from the amplified RNA of the captured cells, and the specific immobilized DNA molecules on the microarray. The hybridization results demonstrate, for example, which genes of those represented on the microarray as probes, are hybridized to cDNA from the captured cells, and/or the amount of specific gene expression. The hybridization results represent the gene expression profile of the captured cells. The gene expression profile of the captured cells can be used to compare with the gene expression profile of a different set of captured cells, and the similarities and differences provide useful information for determining the differences in gene expression between different cell types, and differences between the same cell type under different conditions.

Accordingly, the present invention provides the methods of the appended claims.

### BRIEF DESCRIPTION OF THE DRAWINGS

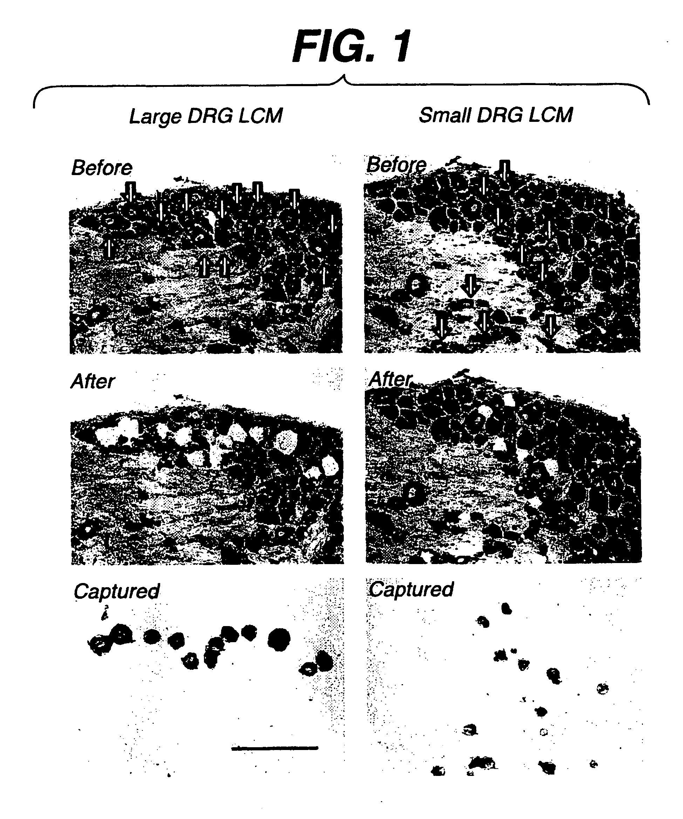

Figure 1 - Laser capture microdissection (LCM) from 10 µm Nissl-stained sections of adult rat large and small dorsal root ganglion (DRG) neurons is shown. The red arrows indicate DRG neurons to be captured (top panel). The middle and bottom panels show successful capture and film transfer respectively. Scale bar = 200 µm,

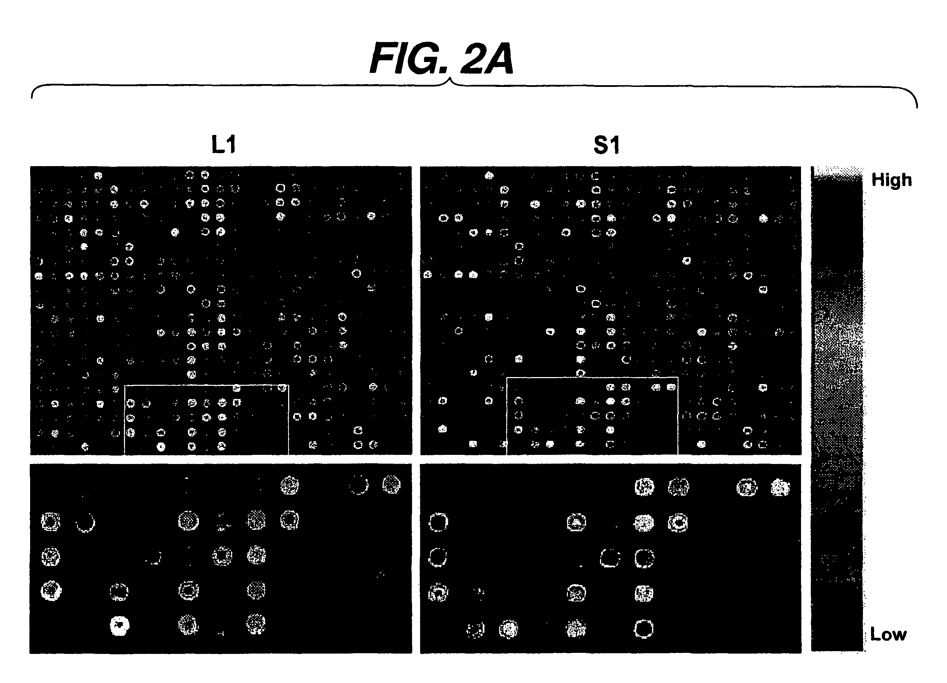

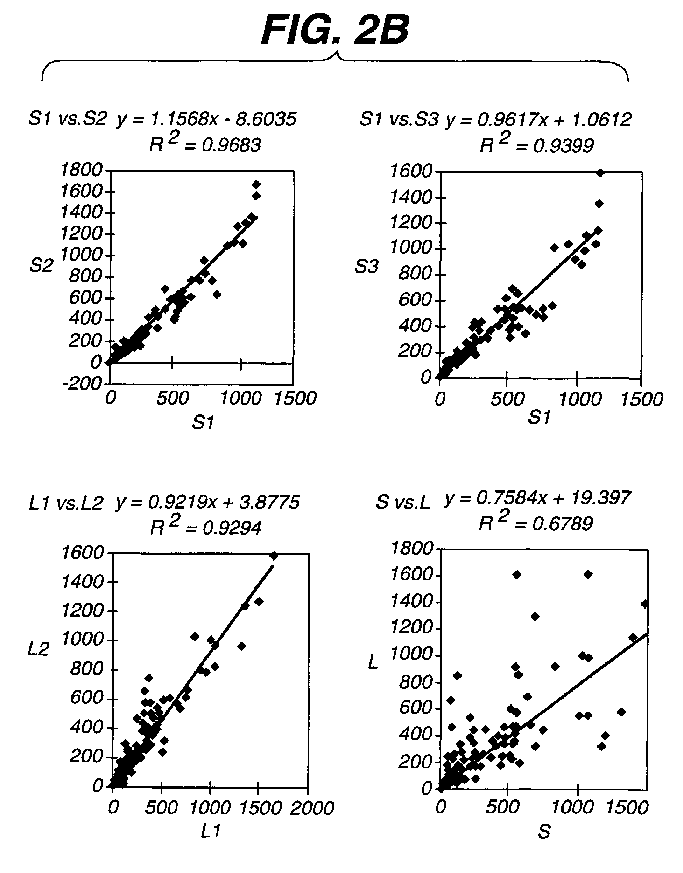

Figure 2, Panels A and B - cDNA microarray expression patterns of small (S) and large (L) neurons is shown. In 2A is an example of the cDNA micraoarray data obtained. Boxed in white is an identical region of the microarray for L1 and S1 samples that is enlarged (shown directly below). In 2B, scatter plots showing correlation between independent amplifications of S 1 vs. S2, S1 vs. S3, L 1 vs. L2 and L (L and L2) vs. S (S1, S2 and S3).

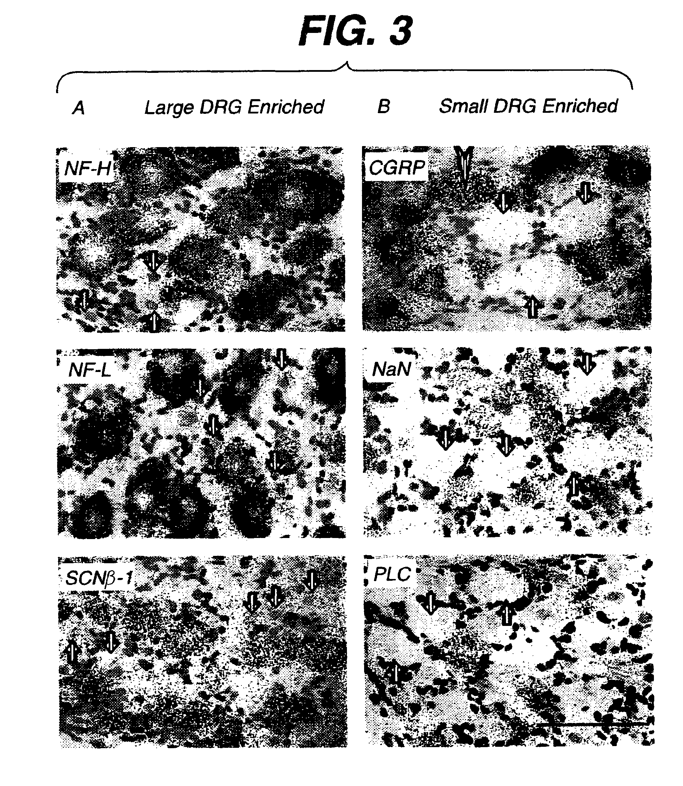

Figure 3, Panels A and B - Representative fields of radioisotopic *in situ* hybridization of rat DRG with selected cDNAs is shown. The sections were Nissl-counterstained. The left panel (panel A) shows results with radiolabled probes encoding neurofilament-high (NF-H), neurofilament-low (NF-L) and β-1 subunit of the voltage-gated sodium channel (SCNβ-1). Red arrows in the left panel denote identifiable small neurons. The right panel (panel B) shows representative fields from radiolabeled probes encoding calcitonin generelated product (CGRP), voltage gated Na channel (NaN) and phospholipase C delta 4 (PLC). Red arrows in the right panel denote identifiable large neurons. Large red arrowhead denotes a large neuron which is also labeled. Scale bar = 100 µm.

### DETAILED DESCRIPTION OF THE INVENTION

The present invention provides a new method for the generation of gene expression profiles in a specific chosen set of cells. The method of the present invention utilizes known techniques that are combined in a novel way to provide a methodology for accurately isolating only the cells that are desired for the analysis. This is accomplished through laser capture dissection in which individual cells from a mixed population of cells, such as an organ or tissue sample, become attached to a substrate by laser energy applied to specific cells selected for capture. The captured cells are separated from the non-captured cells to provide a set of cells that were specifically selected for inclusion within the set. Selection criteria can be any characteristics of the cells that are desired for inclusion within the set. Using this technique a set of cells is collected that have the desired characteristics, and represent a uniform collection of cells. The captured cells are then used to produce extracted RNA. The RNA is extracted from the captured cells using known techniques. This extracted RNA is then amplified using known techniques to about one million-fold amplification, and the amplified RNA is, optionally, used to produce cDNA using known techniques.

To demonstrate this integration of technologies, the differential gene expression between large and small-sized neurons in the dorsal root ganglia (DRG) was examined. In general, large DRG neurons are myelinated, fast-conducting and transmit mechanosensory information, while small neurons are unmyelinated, slow-conducting and transmit nociceptive information¹². This system was chosen because: (1) numerous differentially expressed genes (small vs. large) have been previously reported; thus the success of this experiment could be assessed and (2) many small and large neurons are adjacent to each other, thus testing whether individual neurons can be cleanly captured.

As shown in Figure 1, large (diameter of >40µm) and small (diameter < 25µm) neurons were cleanly and individually captured via LCM from 10 µm sections of Nissl-stained rat DRGs. For this study, two sets of 1000 large neurons and 3 sets of 1000 small neurons were captured for cDNA microarray analysis.

### RNA amplification is reproducible between individual sets of neurons

RNA was extracted from each set of neurons and linearly amplified an estimated 10⁶-fold via T7 RNA polymerase. Once amplified, three fluorescently labeled probes were synthesized from an individually amplified RNA (aRNA) and hybridized in triplicate to a microarray (a.k.a. chip) containing 477 cDNAs (see chip design described below) plus 30 cDNAs encoding plant genes (for the determination of non-specific nucleic acid hybridization). Expression in each neuronal set (designated as S1, S2, and S3 for small and L1 and L2 for large neurons) was thus monitored in triplicate, requiring a total of 15 microarrays. The quality of the microarray data is exemplified in Figure 2A which shows pseudo-color arrays, one resulting from hybridization to probes derived from neuronal set S1 and the other from neuronal set L2. In Figure 2A, the enlarged part of the chip shows some differences in fluorescence intensity (i.e., expression levels) for particular cDNAs and demonstrates that spots containing the different cDNAs are relatively uniform in size and that background between spots is relatively low.

To determine whether a signal corresponding to a particular cDNA is reproducible between different chips, for each neuronal set, the coefficient of variation was calculated (CV or standard deviation/mean X 100%). From these values, the overall average CV for all 477 cDNAs per neuronal set was calculated to be: 15.81%=S1, 16.93% =S2, 17.75% = S3, 20.17% = L1 and 19.55% = L2.

More importantly, independent amplifications (~10⁶-fold) of different sets of the same neuronal subtype yielded quite similar expression patterns. For example, the correlation of signal intensities between S 1 vs. S2 was R² = 0.9688, and between S 1 vs. S3 was R² = 0.9399 (Figure 2B). Similar results were obtained between the two sets of large neurons:

R²= 0.929 for L1 vs. L2 (Figure 2B).

Conversely, a comparison between all three small neuronal sets (S1, S2 and S3) versus the two large sets (Ll and L2) yielded a much lower correlation (R² = 0.6789), demonstrating as expected that a subset of genes are differentially expressed between the two neuronal subtypes (Figure 2B).

### Differential gene expression is demonstrated between small and large neurons

To identify the mRNAs that are differentially expressed between large and small neurons, all 477 cDNAs were examined and those with 1.5-fold or greater differences (at P<0.05) were sequenced and are shown in Table 1 and 2. Amongst the collection of cDNAs on the microarray, it was found that more mRNAs preferentially expressed in small neurons (14 mRNAs in large versus 27 in small); this may simply reflect the set of cDNAs used on this chip.

To confirm the observed differential gene expression, *in-situ* hybridization was performed with a subset of these cDNAs.

For small neurons, five mRNAs were examined which encoded the following: fatty acid binding protein (GenBank accession # M13501), NaN (sodium voltage-gated channel, AF059030), phospholipase C delta-4 (U16655), CGRP (L00111) and annexin V (82462).

All five mRNAs are preferentially expressed in small neurons (three of the five are shown in Figure 3, see Table 3 for all five). This was based on quantitative measurements in which was measured for a given mRNA the (1) overall intensity of signal in small and large neurons and (2) percentage of cells labeled within the total population of either small or large neurons (Table 3).

The results confirmed *in-situ* hybridization studies for NaN mRNA 13 and CGRP mRNA¹⁴ and are consistent with immunofluorescent studies with annexin V¹⁵.

Phospholipase C delta 4 has previously been shown to be induced in S-phase of the cell cycle and reside in the nucleus¹⁶. Given that neurons are post-mitotic, this observation suggests that this enzyme may play a different role in a subset of small neurons.

For large neurons, three cDNAs were examined, neurofilaments NF-L (M25638) and NF-H (J04517) as well as the beta-1 subunit (M91808) of voltage-gated sodium channels. As shown in Figure 3 and Table 3, preferential expression of these mRNAs was found in large DRG neurons. Recent *in-situ* hybridization studies have also demonstrated preferential expression in large neurons for these three mRNAs.^{14 17} In addition, previous *in-situ* hybridization studies are in agreement with this cDNA chip data for the differential expression in small and large neurons of P2X3 receptor mRNA (X90651), NF-middle (J04517), hsp 27 ¹⁸and peripherin (M26232).^{14, 19, 20}. One report, however, finds no differences between small and large neurons for peripherin mRNA expression.¹⁴

In general, for a given mRNA, the cDNA chip data and the results from the *in-situ* hybridization studies are in agreement. However, in most cases, *in-situ* hybridization studies indicate much greater differences in expression between small and large than what is observed from cDNA chip data (Table 1 and 2 vs. Table 3). This is particularly apparent for low intensity signals. For example, phospholipase C delta 4 expression was found almost exclusively in small neurons (Table 3) although the chip data indicates only a 2.76-fold difference in expression (Table 2). In most part, this can be explained by the fact that background signal due to non-specific nucleic acid hybridization (i.e., hybridization signal from plant cDNAs) has not been subtracted from each cDNA intensity. As indicated below, the 75-percentile value background signal for plant cDNAs is 48.68 for small and 40.94 for large neuronal sets. Thus the ratio of expresion of small to large neurons for phospholipase C delta 4 would go from a 2.76-fold difference to ~22-fold difference if the "non-specific" background is subtracted. The reason for not subtracting this background is that it can also lead to very large and potentially spurious fold-differences as the denominator (i.e., intensity of signal minus 75% plant value) approaches zero.

Overall, of the 41 mRNAs preferentially expressed in either large or small neurons, similar results have been demonstrated for 12 of these mRNAs via *in-situ* hybridization studies.

This level of success suggests that most of the other 30 mRNAs are also differentially expressed in small or large neurons.

### Building gene expression databases containing cell type specificity

It was demonstrated that by integrating LCM, aRNA and cDNA chips, one can successfully screen different cell types obtained from *in-situ* and subsequently identify differential gene expression. Although this study has identified mRNAs with differential expression within DRG neurons, there exists a great deal more heterogeneity within DRG neurons beyond simply small and large. For example, within small neurons (i.e., nociceptive neurons) there is heterogeneity with respect to gene expression, which presumably reflects, at least in part, the different sensory modalities transmitted. To approach this more complicated heterogeneity, the coupling of immunocytochemistry to LCM followed by aRNA and DNA chip analysis can be done. In addition, chips containing a larger number of cDNAs (i.e., >10,000) can be completed to more fully identify differential gene expression between large and small neurons.

The results shown herein demonstrate that expression profiles generated via this integration of known technologies can not only be useful for screening cDNAs, but also, more importantly, to produce databases that contain cell type specific gene expression.

Cell type specificity within a database will give an investigator much greater leverage in understanding the contributions of individual cell types to a particular normal or disease state and thus allow for a much finer hypotheses to be subsequently generated.

Furthermore, genes, which are coordinately expressed within a given cell type, can be identified as the database grows to contain numerous gene expression profiles from a variety of cell types (or neuronal subtypes). Coordinate gene expression may also suggest functional coupling between the encoded proteins and therefore aid in one's attempt to determine function for the vast majority of cDNAs currently cloned.

### Table 1

mRNA enriched in large DRG neurons. [GB] gene bank accession number; [Mean] mean intensity of DNA chip microarrays; [S.E.M.] standard error of mean; [Ratio] mean intensity ratio of large DRG vs. small DRG neurons; [*] mean intensity not significantly different (p >0.05) from 75% of plant value.

### Table 2

mRNA enriched in small DRG neurons. [GB] gene bank accession number; [Mean] mean intensity of DNA chip microarrays; [S.E.M.] standard error of mean; [Ratio] mean intensity ratio of small DRG vs. large DRG neurons; [*] mean intensity not significantly different (p >0.05) from 75% of plant value.

### Table 3

In situ hybridization of selected cDNA clones. [Intensity] = estimated mRNA expression level per cell as follows: [-] no above background expression; [±] weak expression; [+] mild expression; [++] moderate expression; [+++] strong expression. [%] = percentage of DRG neurons expressing above background the mRNA of interest.

**Table 1**

| | | | Small DRG | | Large DRG | |
|---|---|---|---|---|---|---|
| Clone ID | GB | Description | intensity | % of Labeled | intensity | % of Labeled |
| 192393 | M25638 | Rat smallest neurofilament protein (NF-L) | ± | 100% | +++ | 100% |
| 192157 | J04517 | Rat high molecular weight neurofilament (NF-H) | ±/- | 21.40% | +++ | 98.60% |
| 192424 | M91808 | Rattus norvegicus sodium channel beta-1 | ± /- | 10% | ++ | 96.30% |
| 192273 | M13501 | Rat liver fatty acid binding protein, | +/++ | 62.20% | +/- | 1% |
| 192294 | AF059030 | Rattus norvegicus voltage-gated Na channel NaN | ++/+ | 96.70% | +/- | 4.20% |
| 192199 | D42137 | Rat annexin V gene | + / ++ | 95.00% | + / ++ | 74.00% |
| 192207 | U16655 | Rattus norvegicus phospholipase C delta-4 | ++ | 42.20% | - | 0% |
| 191857 | L00111 | Rat CGRP | +++/++ | 83.70% | ++/- | 9.40% |

**Table 2**

| | | | | | | |
|---|---|---|---|---|---|---|
| 192294 | AF059030 | Rattus norvegicus voltage-gated Na channel alpha subunit NaN | 161.34±20.07 | 51.3±12.99* | 3.15 | 0.0005 |
| 192195 | D86642 | Rat mRNA for FK506-binding protein | 496,33±40.11 | 158.8±35.13 | 3.13 | 0.0005 |
| 192207 | U16655 | Rattus norvegicus phospholipase C delta-4 | 146.33±10.03 | 53.06±4.23 | 2.76 | 0.0005 |
| 192163 | X90651 | R. norvegicus P2X3 receptor | 390.28±10.4 | 164.81±26.22 | 2.37 | 0.0005 |
| 191858 | S69874 | C-FABP=cutaneous fatty acid-binding protein [rat) | 448.26±30.01 | 196.97±18.68 | 2.28 | 0.0005 |
| 192139 | D45249 | Rat proteasome activator rPA28 subunit alpha | 104.46±5.24 | 47.74±6.97* | 2.19 | 0.0005 |
| 192178 | L12447 | Mus musculus insulin-like growth factor binding protein 5 | 288.97±8.47 | 141.67±5.61 | 2.04 | 0.0005 |
| 192306 | X77953 | R.norvegicus ribosomal protein S15a. | 415.77±54-08 | 204.19±25.03 | 2.04 | 0.005 |
| 192129 | M38188 | Human unknown protein from clone pHGR74 | 114.72±10.98 | 57.47±11.64* | 2.00 | 0.0025 |
| 192339 | | Novel | 83.94±6.26 | 42.42±7.75* | 1.98 | 0.001 |
| 191857 | L00111 | Rat CGRP | 900.1±45.83 | 459.99±35.39 | 1.96 | 0.0005 |
| 192203 | AF059486 | Mus musculus putative actin-binding protein DOC6 | 861.16±32.58 | 448.32±68.77 | 1.92 | 0.0005 |
| 192351 | U25844 | Mus musculus serine proteinase inhibitor (SPI3) | 271.95±30.44 | 142.81±6.93 | 1.90 | 0.0025 |
| 191837 | M29472 | Rattus norvegicus mevalonate kinase | 94.44±9.63 | 51.83±5.95* | 1.82 | 0.0025 |
| 191628 | | Novel | 635.92±73.01 | 363.86±11.53 | 1.75 | 0.005 |
| 192175 | | Novel | 181.28±13.23 | 105.36±10.39 | 1.72 | 0.0005 |
| 192284 | | Novel | 188.28±13 | 110.53±7.27 | 1.70 | 0.0005 |
| 192330 | Y10386 | MMC1INH M.musculus C1 inhibitor | 134.88±11.01 | 79.3±5.51 | 1.70 | 0.0005 |
| 192199 | D42137 | Rat annexin V gene | 439.57±13.62 | 265.21±14.97 | 1.66 | 0.0005 |
| 192011 | M98194 | Rat extracellular signal-regulated kinase 1 | 319.35±32.79 | 194.88±6.83 | 1.64 | 0.005 |
| 192206 | U59673 | Rattus norvegicus 5HT3 receptor | 139.96±4.07 | 85.48±6.17 | 1.64 | 0.0005 |
| 192167 | U23146 | Rattus norvegicus mitogenic regulation SSeCKS | 456.44±13.34 | 300.71±23.25 | 1.52 | 0.0005 |
| 191848 | M93056 | Human mononcyte/neutrophil elastase inhibitor | 125.16±14.76 | 82.56±15.38 | 1.52 | 0.05 |
| 192309 | | Novel | 463.17±45.37 | 308.05±25.45 | 1.50 | 0.01 |

**Table 3**

| PRI ID | GB | Description | Mean±S.E.M.( **Small)** | Mean±S.E.M. **(Large)** | Ratio | p |
|---|---|---|---|---|---|---|
| 192393 | M25638 | Rat smallest neurofilament protein (NF-L) | 63.3±6.12 | 551.56±34.94 | 8.71 | 0.0005 |
| 191624 | M14656 | Rat osteopontin | 53.4±4.11* | 218.52±22.81 | 4.09 | 0.0005 |
| 192157 | J04517 | Rat high molecular weight neurofilament (NF-H) | 475.86±18.59 | 1319.77±50.3 | 2.77 | 0.0005 |
| 192282 | Z12152 | R.norvegicus neurofilament protein middle | 75.93±3.75 | 206.55±9.92 | 2.72 | 0.0005 |
| 192378 | D87445 | Human KIAA0256 | 30.26±2.66* | 77.42±17.52 | 2.56 | 0.025 |
| 192283 | | Novel | 50.9±3.45* | 128.56±6.86 | 2.53 | 0.0005 |
| 192125 | V00681 | R.norvegicus mitochondrial genes for 16S rRNA, tRNA | 186.5±14.61 | 445.82±23.95 | 2.39 | 0.0005 |
| 191851 | X51396 | Mouse MAP1B microtubule-associated protein | 90.84±5.91 | 215.55±21.35 | 2.37 | 0.0025 |
| 192424 | M91808 | Rattus norvegicus sodium channel beta-1 | 83.99±7.93 | 194.88±20.61 | 2.32 | 0.0025 |
| 191862 | S67755 | hsp 27=heat shock protein 27 [rats, Sprague-Dawley) | 144.74±10.14 | 265.94±19.44 | 1.84 | 0.0005 |
| 192016 | L10426 | Mus musculus ets-related protein 81 (ER81) | 43.85±1.89* | 80.04±7.16 | 1.83 | 0.0025 |
| 192228 | | Novel | 28.9±1.11* | 52±3.41 | 1.80 | 0.0005 |
| 192411 | M21551 | Human neuromedin B | 57.62±5.56* | 97.18±6.61 | 1.69 | 0.0005 |
| 192422 | | Novel | 110.06±11.78 | 168.52±12.14 | 1.53 | 0.0025 |

### EXAMPLE 1

**Laser Capture Microdissection (LCM).** Two adult female Sprague Dawley rats were used in this study. Animals were anesthetized with Metofane (Methoxyflurane, Cat# 556850, Mallinckrodt Veterinary Inc. Mundelein, IL, USA) and sacrificed by decapitation. Using RNase-free conditions, cervical dorsal root ganglia (DRGs) were quickly dissected out, placed in cryomolds, covered with frozen-tissue embedding medium OCT (Tissue-Tek), and frozen in dry ice-cold 2-methylbutane (~ -60 °C). The DRGs were then sectioned at 7-10 µm in a cryostat, mounted on plain (non-coated) clean microscope slides and immediately frozen on a block of dry ice. The sections were stored at -70 °C until further use.

A quick Nissl (cresyl violet acetate) staining was employed in order to identify the DRG neurons. This was completed as follows. Slides containing sections were quickly loaded on a slide holder, immediately fixed in 100% ethanol for 1 minute followed by rehydration via subsequent steps of 95%, 70%, 50% ethanol diluted in RNase free deionized H₂O (5 seconds each). Next, the slides were stained with 0.5% Nissl/0.1 M sodium acetate buffer for 1 minute, dehydrated in graded ethanols (5 seconds each) and cleared in xylene (1 min.). Once air-dried, the slides were ready for LCM.

The PixCell II LCM™ System from Acturus Engineering Inc. (Mountain View, CA) was used for laser-capture. Following manufacture's protocols, 2 sets of large and 3 sets small DRG neurons (1000 cells per set) were laser-captured. The criteria for large and small DRG neurons are as follows: a DRG neuron was classified as small if it had a diameter <25 µm plus an identifiable nucleus whereas a DRG neuron with a diameter >40 µm plus an identifiable nucleus was classified as large.

### EXAMPLE 2

**RNA Extraction of LCM samples**. Total RNA was extracted from the LCM samples with Micro RNA Isolation Kit (Stratagene, San Diego, CA) with some modifications.

Briefly, after incubating the LCM sample with 200 µl of denaturing buffer and 1.6 µl β-ME at room temperature for 5 min., the LCM sample was extracted with 20 µl of 2M sodium acetate, 220 µl phenol and 40 µl chloroform: isoamyl alcohol. The aqueous layer was collected and then mixed with I µl of 10 mg/ml carrier glycogen, and precipitated with 200 µl of isopropanol. Following 70% ethanol wash and air-dry, the pellet was resuspend in 16 µl of RNase free H2O, 2 µl 10X DNase I reaction buffer, 1 µl RNasin and 1 µl of DNase I, incubated at 37°C for 30 minutes to remove any genomic DNA contamination. Next, the phenol chloroform extraction was repeated as above. The pellet was resuspend in 11 µl of RNase free H2O, 1 µl of which was saved and used as a negative control for reverse transcription PCR (no RT control), and the remaining (10 µl) was processed for RT-PCR and RNA amplification.

### EXAMPLE 3

**Reverse Transcription(RT) of RNA.** First stand synthesis was completed by adding together 10 µl of purified RNA from above and 1 µl of 0.5 mg/ml T7-oligodT primer (5'TCTAGTCGACGGCCAGTGAATTGTAATACGACTCACTATAGGGCGT₂₁-3'). Primer and RNA were incubated at 70°C 10 minutes, followed by 42°C for 5 minutes. Next, 4 µl of 5X first strand reaction buffer, 2 µl 0.1M DTT, 1 µl 10mM dNTPs, 1 µl RNasin and 1 µl Superscript II (Gibco BRL) were added and incubated at 42°C for one hour. Next, 30 µl second strand synthesis buffer, 3 µl 10 mM dNTPs, 4 µl DNA Polymerase I, 1 µl *E*. *coli* RNase H, 1 µl *E*. *coli* DNA Ligase and 92 µl of RNase free H₂O were added and incubated at 16°C for 2 hours, followed by 2 µl of T4 DNA Polymerase at 16°C for 10 minutes. Next, the cDNA was phenol-chloroform-extracted and washed 3X with 500 µl of H₂O in a Microcon-100 column (Millipore). After collection from the column, the cDNA was dried down to 8 µl for in-vitro transcription.

### EXAMPLE 4

**T7 RNA Polymerase Amplification (aRNA)**. *Ampliscribe* T7 Transcription Kit (Epicentre Technologies) was used: 8 µl double-stranded cDNA, 2 µl of 10X *Ampliscribe* T7 buffer, 1.5 µl of each 100 mM ATP, CTP, GTP and UTP, 2 µl 0.1 M DTT and 2 µl of T7 RNA Polymerase, at 42°C for 3 hours. The aRNA was washed 3X in a Microcon-100 column, collected, and dried down to 10 µl.

**Subsequent Rounds of aRNA Amplification.** 10 µl of aRNA from first round amplification was mixed together with 1 µl of 1mg/ml random hexamers (Pharmacia), 70°C for 10 minutes, chilled on ice, equilibrated at room temperature for 10 minutes, then 4 µl 5X first stand buffer, 2 µl 0.1M DTT, 1 µl 10mM dNTPs, 1 µl RNasin and 1 µl Superscript RT II were added and incubated at room temp. for 5 minutes followed by 37°C for 1 hour. Then, 1 µl of RNase H was added and incubated at 37°C for 20 min. For second strand cDNA synthesis, 1 µl of 0.5 mg/ml T7-oligodT primer was added and incubated at 70°C for 5 minutes, 42°C for 10 minutes. Next, 30 µl of second strand synthesis buffer, 3 µl 10mM dNTPs, 4 µl Polymerse I, 1 µl E. *coli* RNase H, 1 µl E. *coli* DNA Ligase and 90 µl of RNase free H2O were added and incubated at 37°C for 2 hours. Then 2 µl of T4 DNA Polymerase was added at16°C for 10minutes. The double strand of cDNA was extracted with 150 µl of phenol chloroform to get ride of protein and purified with Microcon-100 column (Millipore) to separate from the unincorporated nucleotides and salts. The cDNA is ready for second round T7 in vitro transcription as above and then a subsequent third round aRNA amplification.

### EXAMPLE 5

**Microarray Design.** The cDNAs present on the chip were obtained from two separate differential display²¹ experiments. First, we preformed a screen to clone mRNAs preferentially expressed in DRG versus brain, kidney and liver. Second, a screen was done to identify/clone mRNAs with decreased or increased concentration in ipslateral ("treated") lumbar 5 and 6 DRGs that were tightly ligated distal to the dorsal root ganglion ("Chung" model²²) versus contralateral ("control") lumbar 5 and 6 DRGs.

**Microarray Printing.** 477 clones in vector PCR 2.1 from our previous differential display studies (as described above) were printed on silylated slides (CEL Associates). cDNAs were PCR-amplified with 5' amino-linked primers and purified with Qiagen 96 PCR Purification Kits. The print spots were about 125 µm in diameter and were spaced 300 µm apart from center to center. 30 plant genes were also printed on the slides as a control for non-specific hybridization (gift from Mark Schena)

### EXAMPLE 6

**Microarray Probe Synthesis.** Cy3 labeled cDNA probes were synthesized from aRNA of LCM DRGs with Superscript Choice System for cDNA Synthesis (Gibco BRL). In brief, 5 µg aRNA, 3 µg random hexamer were mixed in a total volume of 26 µl (containing RNase free H₂O), heated to 70°C for 10 minutes and chilled on ice. Then,10 µl first strand buffer, 5 µl 0.1MDTT, 1.5 µl RNasin. 1 µl 25mMd(GAT)TP, 2 µl 1mM dCTP, 2 µl Cy3-dCTP (Amersham) and 2.5 µl Superscript RT II were added and incubated at room temp. for 10 minutes and then 37°C for 2 hours. To degrade the aRNA template, 6 µl 3N NaOH was added and incubated at 65°C for 30 minutes. Then, 20 µl 1M Tris-HCl pH 7.4, 12 µl 1N HCl and 12 µl H₂O were added. The probes were purified with Microcon 30 Columns (Millipore) and then with Qiagen Nucleotide Removal Columns. The probes were vacuum dried and resuspend in 20 µl of hybridization buffer (5X SSC, 0.2% SDS) containing mouse Cot1 DNA (Gibco BRL).

**Microarray Hybridization and Washes**. Printed glass slides were treated with sodium borohydrate solution ( 0.066M NaBH4, 0.06M Na AC ) to ensure amino-linkage of cDNAs to the slides. Then, the slides were boiled in water for 2 minutes to denature the cDNA. Cy3 labeled probes were heated to 99°C for 5minutes, room temperature for 5 minutes and applied to the slides. The slides were covered with glass cover slips, sealed with DPX (Fluka) and hybridized at 60°C for 4-6 hours. At the end of hybridization slides were cooled to room temperature. The slides were washed in 1X SSC, 0.2% SDS at 55°C for 5 minutes, 0.1X SSC, 0.2% SDS at 55°C for 5 minutes. After a quick rinse in 0.1X SSC, 0.2% SDS, the slides were air-blown dried and ready for scanning.

**Microarray Quantitation.** cDNA microarrays (i.e., microscope slides) were scanned for cy3 fluorescence using the ScanArray 3000 (General Scanning, Inc.). ImaGene Software (Biodiscovery, Inc.) was then subsequently used for quantitation. In total, 15 chips were processed, with 3 chips/neuronal set (see text). Briefly, the intensity of each spot (i.e., cDNA) was corrected by subtracting the immediate surrounding background. Next, the corrected intensities were normalized for each cDNA (i.e., spot) with the following formula: intensity (background corrected) / 75-percentile value of the intensity of the entire chip x 1000. To determine "non-specific" nucleic acid hybridization, 75-percentile values were calculated from the individual averages of each plant cDNA (for a total of 30 different cDNAs) from each neuronal set. The overall 75-percentile value for S1, S2 and S3 = 48.68 and for L1 and L2 = 40.94.

**Statistical Analyses.** To assess correlation of intensity value for each cDNA between individual sets of neurons (e.g., S1 vs. S2 in Fig. 2B) or between two neuronal subtypes (i.e., S1, S2 and S3 vs. L1 and L2 in Fig. 2B), scatter plots were used and linear relationships were measured. The coefficient of determination, R², that was calculated, indicates the variability of intensity values in one group vs. the other.

To statistically determine whether or not intensity values measured from microarray quantitation are true signals, each intensity is compared, via a one-sample t-test, to the 75-percentile value of 30 plant cDNAs that are present on each chip (representing non-specific nucleic acid hybridization). Values not significantly different from the 75-percentile value that are in presented in Table 1 and 2 and so denoted. To determine which cDNAs are statistically significant in their differential gene expression between large and small neurons, the intensity for each cDNA from neuronal sets for large neurons (L1 and L2) and small neurons (S1,S2, and S3) were grouped together respectively and intensity values were averaged for each corresponding cDNA. Two-sample *t* test for one-tailed hypotheses was used to detect a gene expression difference between small neuron and large neurons.

### EXAMPLE 7

***In Situ* Hybridization.** *In situ* hybridization was carried out as previously described.²³ Briefly, cDNAs were subcloned into pBluescript II SK (Stratagene) linearized and ³⁵S-UTP was incorporated via in-vitro transcription with T7 or T3 RNA polymerase. The probes were then purified with Quick Spin™ Columns( Boehringer Mannheim). Probes (10⁷ cpm's /probe) were hybridized to 10 µm, 4% paraformaldehyde-fixed rat DRG sections which were mounted on Superfrost Plus slides (VWR). After overnight hybridization at 58°C and post-washes, the slides were exposed to film for primary data.

Subsequently the slides were coated with Kodak liquid emulsion NTB2 and exposed in light-proof boxes for 1-2 weeks at 4°C. The slides were developed in Kodak Developer D-19, fixed in Kodak Fixer and Nissl stained for expression analysis.

Under light field microscopy, mRNA expression levels of specific cDNAs were semi-quantitatively analyzed. This was done as follows: no expression (-, grains were <5 fold of the background); weak expression (±, grains were 5-10 fold of the background); low expression (+, grains were 10-20 fold of the background); moderated expression (++, grains were 20-30 fold of the background); strong expression (+++, grains were >30 fold of the background). The percentage of small or large neurons expressing a specific mRNA was obtained by counting the number of labeled (above background) and unlabeled cells from either large or small neurons from four sections (at least 200 cells were counted).

### BIBLIOGRAPHY

1. Shalon, D. Gene expression micro-arrays: a new tool for genomic research. *Pathol. Biol.* **46**, 107-109 (1998).
2. Lockhart, D.J. *et al.* Genomics and DNA chips. *Nucleic Acids Symp. Ser.* **38,** 11-12 (1998).
3. Schena, *M. et al.* Microarrays: biotechnology's discovery platform for functional genomics. *Trends Biotechnol.* **16, 301-306** (1998).
4. DeRisi, J.L., Iyer, V.R. & Brown, P.O. Exploring the metabolic and genetic control of gene expression on a genomic scale. *Science (Washington, D. C.)* **278,** 680-686 (1997).
5. Cho, R.J. *et al.* A genome-wide transcriptional analysis of the mitotic cell cycle. *Mol. Cell* **2,** 65-73 (1998).
6. Schena, M. *et al.* Parallel human genome analysis: microarray-based expression monitoring of 1000 genes. *Proc. Natl. Acad. Sci. U. S. A.* **93,** 10614-10619 (1996).
7. Heller, R.A. *et al.* Discovery and analysis of inflammatory disease-related genes using cDNA microarrays. *Proc. Natl. Acad. Sci. U. S. A.* **94,** 2150-2155 (1997).
8. Welford, S.M. *et al.* Detection of differentially expressed genes in primary tumor tissues using representational differences analysis coupled to microarray hybridization. *Nucleic Acids Res.* **26**, 3059-3065 (1998).
9. Emmert-Buck, M.R. *et al.* Laser capture microdissection. *Science (Washington, D.* C.) **274**, 998-1001 (1996).
10. Van Gelder, R.N. *et al.* Amplified RNA synthesized from limited quantities of heterogeneous cDNA. *Proc. Natl. Acad. Sci. U. S. A.* **87**, 1663-7 (1990).
11. Schena, M., Shalon, D., Davis, R.W. & Brown, P.O. Quantitative monitoring of gene expression patterns with a complementary DNA microarray. *Science (Washington, D. C.)* **270**, 467-70 (1995).
12. Coggeshall, W.D.W.a.R.E. *Sensory Mechanisms of the Spinal Cord,* (Plenum Press, New York, 1991).
13. Dib-Hajj, S.D., Tyrrell, L., Black, J.A. & Waxman, S.G. NaN, a novel voltage-gated Na channel, is expressed preferentially in peripheral sensory neurons and down-regulated after axotomy. *Proc. Natl. Acad. Sci. U. S. A.* **95,** 8963-8968 (1998).
14. Goldstein, M.E., Grant, P., House, S.B., Henken, D.B. & Gainer, H. Developmental regulation of two distinct neuronal phenotypes in rat dorsal root ganglia. *Neuroscience (Oxford)* 71, 243-5 8 (1996).
15. Naciff, J.M., Kaetzel, M.A., Behbehani, M.M. & Dedman, J.R. Differential expression of annexins I-VI in the rat dorsal root ganglia and spinal cord. *J. Comp. Neurol.* **368**, 356-370 (1996).
16. Liu, N., Fukami, K., Yu, H. & Takenawa, T. A new phospholipase C .delta.4 is induced at S-phase of the cell cycle and appears in the nucleus. *J. Biol. Chem.* **271**, 355-60 (1996).
17. Oh, Y., Sashihara, S., Black, J.A. & Waxman, S.G. Na+ channel .beta.1 subunit mRNA: differential expression in rat spinal sensory neurons. *Mol. Brain Res. 30,* 357-61 (1995).
18. Costigan, M. *et al.* Heat shock protein 27: developmental regulation and expression after peripheral nerve injury. *J. Neurosci.* **18**, 5891-5900 (1998).
19. Linda M. Parysek, R.L.C., Catherine A. Ley, and Robert D. Goldman. A Type III Intermediate Filament Gene Is Expressed in Mature Neurons. *Neuron* **1**, 395-401 (1988).
20. Nicke, A*. et al.* P2X1 and P2X3 receptors form stable trimers: a novel structural motif of ligand-gated ion channels. *EMBO J.* **17**, 3016-3028 (1998).
21. Liang, P. & Pardee, A.B. Differential display of eukaryotic messenger RNA by means of the polymerase chain reaction. *Science (Washington, D. C., 1883-)* **257**, 967-71(1992).
22. Kim, S.H.a.C., J.M.,. An experimental model for peripheral neuropathy produced by segmental spinal nerve ligation in the rat. *Pain* **50***,* 355-363 (1992).
23. Simmons, D.M., Arriza, J.L. & Swanson, L.W. A complete protocol for in situ hybridization of messenger RNAs in brain and other tissues with radiolabeled single- stranded RNA probes. *J. Histotechnol.* **12**, 169-81 (1989).

## Claims

1. A method, comprising:
a) selecting and attaching cells to a substrate by laser capture microdissection, and isolating the captured cells from the remaining cells;
b) extracting the RNA from the isolated captured cells and amplifying the RNA;
c) producing cDNA from the amplified RNA of step b) and labelling the cDNA with a detectable label to produce labeled cDNA;
d) hybridizing the labeled cDNA of step c) with DNA probes on an immobilized DNA microarray; and
e) determining which immobilized DNA on the microarray hybridized with labeled cDNA, quantitatively and/or qualitatively.

2. A method, comprising:
a) selecting and attaching cells to a substrate by laser capture microdissection, and isolating the captured cells from the remaining cells;
b) extracting the RNA from the isolated captured cells and amplifying the RNA and labelling the amplified RNA with a detectable label to produce labeled amplified RNA;
c) hybridizing the labeled amplified RNA of step b) with immobilized DNA on an immobilized DNA microarray; and
d) determining which immobilized DNA on the microarray hybridized with labeled amplified RNA, quantitatively and/or qualitatively.

3. A method, comprising:
a) selecting and attaching cells to a substrate by laser capture microdissection, and isolating the captured cells from the remaining cells;
b) extracting the RNA from the isolated captured cells and amplifying the RNA and labelling the amplified RNA with a detectable label to produce labeled amplified RNA;
c) producing cDNA from the amplified RNA of step b) and labelling the cDNA with a detectable label to produce labeled cDNA;
d) hybridizing the labeled amplified RNA and/or labeled cDNA with DNA on an immobilized DNA microarray; and
e) determining which immobilized DNA on the microarray hybridized with labeled amplified RNA and/or labeled cDNA, quantitatively and/or qualitatively.

## Patentansprüche

1. Ein Verfahren, umfassend:
a) Auswählen und Anheften der Zellen an ein Substrat durch Laser-Fang-Mikropräparation und Isolieren der gefangenen Zellen von den verbleibenden Zellen;
b) Extrahieren der RNA von den isolierten gefangenen Zellen und Amplifizieren der RNA;
c) Herstellen von cDNA von der amplifizierten RNA aus Schritt b) und Markieren der cDNA mit einem nachweisbaren Marker, um markierte cDNA herzustellen;
d) Hybridisieren der markierten cDNA aus Schritt c) mit DNA Sonden auf einem immobilisierten DNA Mikroarray; und
e) quantitative und/oder qualitative Auswertung der immobilisierten DNA auf dem Mikroarray, die mit der markierten cDNA hybridisiert hat.

2. Ein Verfahren, umfassend:
a) Auswählen und Anheften der Zellen an ein Substrat durch Laser-Fang-Mikropräparation und Isolieren der gefangenen Zellen von den verbleibenden Zellen;
b) Extrahieren der RNA von den isolierten gefangenen Zellen und Amplifizieren der RNA und Markieren der amplifizierten RNA mit einem nachweisbaren Marker, um markierte amplifizierte RNA herzustellen;
c) Hybridisieren der markierten amplifizierten RNA aus Schritt b) mit immobilisierter DNA auf einem immobilisierten DNA Mikroarray; und
d) quantitative und/oder qualitative Auswertung der immobilisierten DNA auf dem Mikroarray, die mit der markierten amplifizierten DNA hybridisiert hat.

3. Ein Verfahren, umfassend:
a) Auswählen und Anbringen der Zellen an ein Substrat durch Laser-Fang-Mikropräpäration und Isolieren der gefangenen Zellen von verbleibenden Zellen;
b) Extrahieren der RNA von den isolierten gefangenen Zellen und Amplifizieren der RNA und Markieren der amplifizierten RNA mit einem nachweisbaren Marker, um markierte amplifizierte RNA herzustellen;
c) Herstellen von cDNA aus der amplifizierten RNA aus Schritt b) und Markieren der cDNA mit einem nachweisbaren Marker, um markierte cDNA herzustellen;
d) Hybridisieren der markierten amplifizierten RNA und/oder markierten cDNA mit DNA auf einen immobilisierten DNA Mikroarray; und
e) quantitative und/oder qualitative Auswertung der immobilisierten DNA auf dem Mikroarray, die mit markierter amplifizierter RNA und/oder markierter cDNA hybridisiert hat.

## Revendications

1. Méthode comprenant:
a) sélection et attachement des cellules à un substrat par microdissection avec capture au laser, et isolement des cellules capturées des cellules restantes;
b) extraction de l'ARN des cellules capturées isolées et amplification de l'ARN;
c) production de l'ADNc à partir de l'ARN amplifié de l'étape b) et marquage de l'ADNc avec un marqueur détectable pour produire l'ADNc marqué;
d) hybridation de l'ADNc marqué de l'étape c) avec des sondes d'ADN sur une micro-série d'ADN immobilisés; et
e) détermination de l'ADN immobilisé sur la micro-série qui s'est hybridé avec l'ADNc marqué, quantitativement et/ou et qualitativement;

2. Méthode comprenant:
a) sélection et attachement des cellules à un substrat par microdissection avec capture au laser, et isolement des cellules capturées des cellules restantes;
b) extraction de l'ARN des cellules capturées isolées et amplification de l'ARN et marquage de l'ARN amplifié avec un marqueur détectable pour produire un ARN amplifié marqué;
c) hybridation de l'ARN amplifié marqué de l'étape b) avec l'ADN immobilisé sur une micro-série d'ADN immobilisés;
d) détermination de l'ADN immobilisé sur la microsérie qui s'est hybridé avec l'ARN amplifié marqué, quantitativement et/ou qualitativement.

3. Méthode comprenant:
a) sélection et attachement des cellules à un substrat par microdissection avec capture au laser, et isolement des cellules capturées des cellules restantes;
b) extraction de l'ARN des cellules capturées isolées et amplification de l'ARN et marquage de l'ARN amplifié avec un marqueur détectable pour produire un ARN amplifié marqué;
c) production d'ADNc à partir de l'ARN amplifié de l'étape b) et marquage de l'ADNc avec un marqueur détectable pour produire de l'ADNc marqué;
d) hybridation de l'ARN amplifié marqué et/ou de l'ADNc marqué avec l'ADN sur une microsérie d'ADN immobilisés; et
e) détermination de l'ADN immobilisé sur la micro-série qui s'est hybridé avec l'ARN amplifié marqué et/ou l'ADNc marqué, quantitativement et/ou qualitativement.
